# EUROPEAN PATENT SPECIFICATION

(11) **EP 1 789 412 B1**
(45) Date of publication and mention of the grant of the patent: **07.05.2008**
(21) Application number: 04770711.2
(22) Date of filing: 16.09.2004
(51) Int. Cl.: C07D 405/12

(54) **CRYSTALLINE ALFUZOSIN BASE**
KRISTALLINE ALFUZOSINBASE
BASE D'ALFUZOSINE CRISTALLINE

(43) Date of publication of application: 30.05.2007
(73) Proprietor: Hetero Drugs Limited, Hyderabad 500 018, Andhrapradesh (IN)
(72) Inventor: PARTHASARADHI REDDY, Bandi, Hetero House, Hyderabad 500 018, Andhrapradesh (IN); RATHNAKAR REDDY, Kura, Hetero Drugs Limited R & D, Balanagar, Hyderabad 500 018 (IN); RAJI REDDY, Rapolu, Hetero Drugs Limited R & D, Balanagar, Hyderabad 500 018 (IN); MURALIDHARA REDDY, Dasari, Hetero Drugs Ltd R & D, Balanagar, Hyderabad 500 018 (IN); RAMAKRISHNA REDDY, Matta, Hetero Drugs Ltd R &D, Balanagar, Hyderabad 500 018 (IN)
(74) Representative: Green, Mark Charles
(86) International application number: PCT/IN2004/000292
(87) International publication number: WO 2006/030449

(56) References cited:
- US-A- 4 315 007
- MANOURY ET AL: 'Synthesis and Antihypertensive Activity of a Series of 4-amino-6,7-Dimethoxyquinazoline Derivatives' J.MED.CHEM. vol. 29, January 1986, pages 19 - 25, XP002994726

## Description

### FIELD OF THE INVENTION

The present invention relates to crystalline solid of alfuzosin base and processes for preparation of the said crystalline solid of alfuzosin base.

### BACKGROUND OF THE INVENTION

U.S. Patent No. 4,315,007 disclosed 4-amino-6,7-dimethoxyquinazol-2-yl alkylenediamine derivatives. The compounds are antihypertensive agents. Among them alfuzosin, chemically N-[3-[(4-amino-6,7-dimethoxy-2-quinazolinyl)methylamino]propyl]tetrahydro-2-furancarboxamide is the most important antihypertensive agent. Alfuzosin is represented by the following structure:

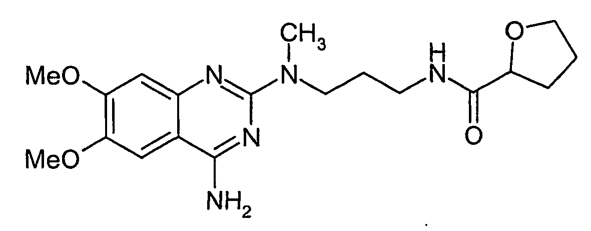

Processes for the preparations of alfuzosin hydrochloride and related compounds were described in U.S. Patent No. 4,315,007 and GB Patent No. 2231571. U.S. Patent No. 5,545,738 disclosed a dihydrate form of alfuzosin hydrochloride, which is also mentioned about the anhydrous, trihydrate and tetrahydrate forms of alfuzosin hydrochloride. The process for the preparation of crystalline solid of alfuzosin base is not disclosed in the prior art. We have discovered that alfuzosin base can be obtained in crystalline solid. Since the crystalline alfuzosin is obtained with high purity, the said crystalline solid can be used to obtain pharmaceutically acceptable salts of alfuzosin in high purity. It has been found that purification of impure alfuzosin base is practically advantageous when compared with the purification of a salt of it.

One object of the present invention is to provide crystalline solid of alfuzosin base.

Another object of the present invention is to provide processes for preparing crystalline alfuzosin base.

Another object of the present invention is to provide purification methods to obtain high purity alfuzosin base and pharmaceutically acceptable salts via crystalline alfuzosin base.

### DETAILED DESCRIPTION OF THE INVENTION

According to one aspect of the present invention, there is provided a process for preparation of crystalline solid of alfuzosin base, the said process comprises stirring a suspension of impure or noncrystalline alfuzosin base in a ketonic solvent or an alcoholic solvent or mixture thereof. The crystalline alfuzosin base may be collected by filtration or centrifugation.

Preferable ketonic solvent is selected from acetone, methyl ethyl ketone, methyl isobutyl ketone, methyl isopropyl ketone and methyl tert-butyl ketone; most preferable ketonic solvents are acetone and methyl isobutyl ketone; preferable alcoholic solvent is selected from methanol, ethanol, isopropyl alcohol and tert-butyl alcohol; and most preferable alcoholic solvents are methanol and ethanol.

Preferably the suspension is stirred for at least 30 minutes at below boiling temperature of the solvent used, more preferably for 1 hour to 4 hours at 25 - 60°C.

According to another aspect of the present invention, there is provided a process for preparation of crystalline solid of alfuzosin base, the said process comprises dissolving alfuzosin base in a ketonic solvent or an alcoholic solvent or mixture thereof and crystallizing alfuzosin base from the solution. The crystalline alfuzosin base may be collected by filtration or centrifugation.

Crystallization may be initiated by a method usually known in the art such as cooling, seeding, partial removal of the solvent from the solution, by adding an anti-solvent to the solution or a combination thereof.

Preferable ketonic solvent is selected from acetone, methyl ethyl ketone, methyl isobutyl ketone, methyl isopropyl ketone and methyl tert-butyl ketone; most preferable ketonic solvents are acetone and methyl isobutyl ketone; preferable alcoholic solvent is selected from methanol, ethanol, isopropyl alcohol and tert-butyl alcohol; and most preferable alcoholic solvents are methanol and ethanol.

According to another aspect of the present invention, there is provided a process for preparation of crystalline solid of alfuzosin base, the said process comprises treating an acid addition salt of alfuzosin with a base to liberate alfuzosin base, isolating by forcible or spontaneous crystallization from a ketonic or alcoholic solvent. The crystalline alfuzosin base may be collected by filtration or centrifugation.

Spontaneous crystallization refers to crystallization without the help of an external aid such as seeding, cooling etc., and forcible crystallization refers to crystallization with the help of an external aid.

Forcible crystallization may be initiated by a method usually known in the art such as cooling, seeding, partial removal of the solvent from the solution, by adding an anti-solvent to the solution or a combination thereof.

The treatment of the acid addition salt with base is carried out in any solvent and the selection of solvent is not critical. A wide variety of solvents such as chlorinated solvents, hydrocarbon solvents, ether solvents etc., may be used.

The base can be inorganic or organic. Preferable base is an inorganic base selected from alkali metal hydroxides, carbonates and bicarbonates. Preferable alkali metal is sodium or potassium.

Preferable ketonic solvent is selected from acetone, methyl ethyl ketone, methyl isobutyl ketone, methyl isopropyl ketone and methyl tert-butyl ketone; most preferable ketonic solvents are acetone and methyl isobutyl ketone; preferable alcoholic solvent is selected from methanol, ethanol, isopropyl alcohol and tert-butyl alcohol; and most preferable alcoholic solvents are methanol and ethanol.

Alfuzosin or a salt thereof used as starting material in the present invention can be prepared by known methods (for example U.S. Patent No. 4,315,007) by reacting N₁-(4-Amino-6,7-dimethoxyquinazol-2-yl)-N₁-methyl propylenediamine with activated tetrahydro-2-furoic acid and optionally converting into the said salt. It has been found that when activated tetrahydro-2-furoic acid is added to N₁-(4-Amino-6,7-dimethoxyquinazol-2-yl)-N₁-methylpropylenediamine, the diamine compound is reacted to a greater extent to form alfuzosin than when the diamine compound is added to activated tetrahydro-2-furoic acid. Therefore, it is advantageous to prepare alfuzosin, which constitutes another aspect of the present invention, by adding activated tetrahydro-2-furoic acid to diamine compound rather than by adding diamine compound to activated tetrahydro-2-furoic acid.

Activated tetrahydro-2-furoic acid refers to tetrahydro-2-furoic acid having its carboxylic acid group in a conventional activated form.

Isolation of alfuzosin base as crystalline solid affords pure alfuzosin, which can be converted into pharmaceutically acceptable salts of alfuzosin. The isolation avoids multiple purification steps of the pharmaceutically acceptable salts of alfuzosin.

In a preferred process, pharmaceutically acceptable salts of alfuzosin such as alfuzosin hydrochloride in pure form can be obtained directly by isolating alfuzosin base as impure product from the reaction mixture, isolating the base as crystalline solid, converting into the salt and isolating the salt formed.

In another preferred process, impure or noncrystalline alfuzosin base is suspended in a ketonic or an alcoholic solvent, stirred for at least 30 minutes at about 25 - 60°C, filtered or centrifuged, the obtained solid is dissolved in an alcoholic or ketonic solvent and crystallized and filtered to give alfuzosin base as crystalline solid.

In another preferred process, an acid addition salt of alfuzosin in impure form is dissolved in an alcoholic or ketonic solvent, a base is added to liberate alfuzosin base and alfuzosin base is isolated as a crystalline solid.

'Impure' in the specification refers to having HPLC purity 95% or less than 95% and 'pure' refers to having HPLC purity more than 95%.

### BRIEF DESCRIPTION OF THE DRAWINGS

Figure 1 is a Differential Scanning Calorimetry of crystalline solid of alfuzosin base.
Figure 2 is an infra-red spectrum of crystalline solid of alfuzosin base.
Figure 3 is a x-ray powder diffraction spectrum of crystalline solid of alfuzosin base.

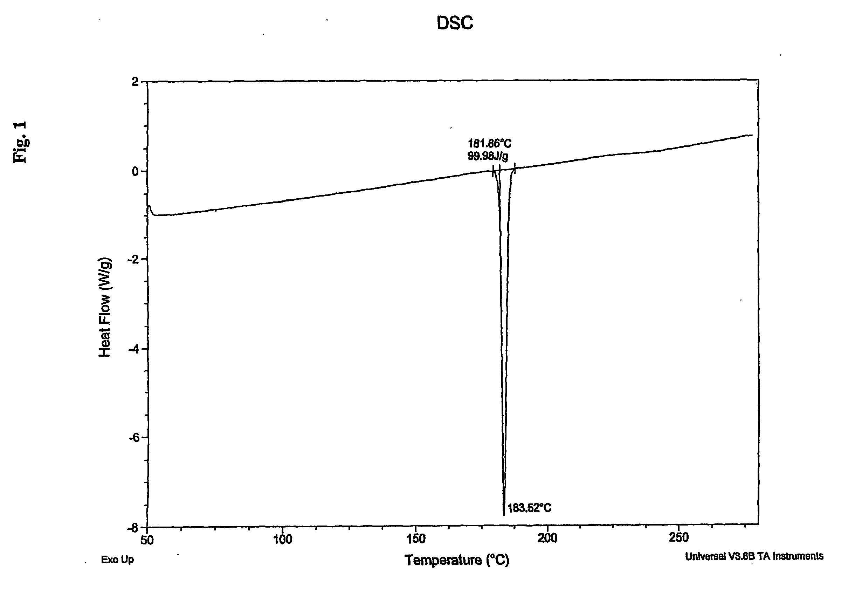

DSC (Differential Scanning Calorimetry) measurements were performed with a DSC Q10 (TA instruments, Inc.). About 3 mg of the powder was placed in an open aluminum pan and it is crimped with an aluminum lid. The crimped sample is then placed in the DSC cell opposite to empty aluminum pan(as reference) and the sample was scanned at 10°C/min from 50°C to 280°C. A typical DSC thermogram of crystalline solid of alfuzosin base is shown in figure 1.

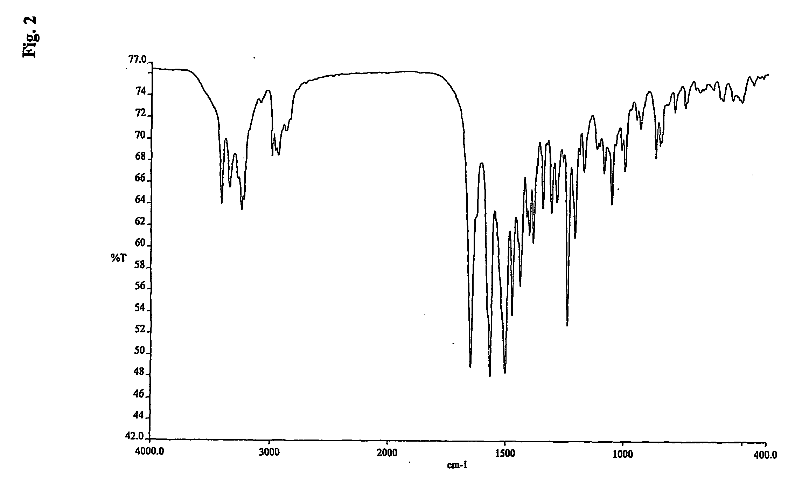

FT-IR spectroscopy was carried out, with a Perkin-Elmer spectrum GX spectrometer. For the production of the KBr compacts approximately 2 mg of sample was powdered with 200 mg of KBr. The spectra were recorded in transmission mode ranging from 4000 to 400 cm⁻¹. A typical infra-red spectrum of crystalline solid of alfuzosin base is shown in figure 2.

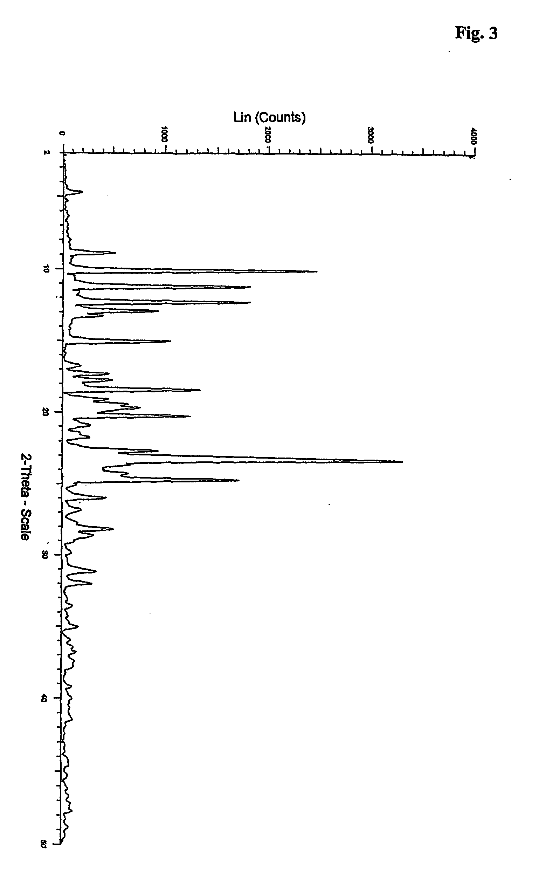

x-Ray powder diffraction spectrum was measured on a Bruker axs D8 advance x-ray powder diffractometer having a Copper-Kα radiation. Approximately 500 mg of sample was gently flattened on a sample holder and scanned from 2 to 50 degrees two-theta, at 0.03 degrees two-theta per step and a step time of 0.5 seconds. The sample was simply placed on the sample holder. The sample was rotated at 30 rpm at a voltage 40 KV and current 35 mA. A typical x-ray powder diffraction spectrum of crystalline solid of alfuzosin base is shown in Figure 3.

The invention will now be further described by the following examples, which are illustrative rather than limiting.

### Comparative example

### Stea-I:

N₁-(4-Amino-6,7-dimethoxyquinazol-2-yl)-N₁-methylpropylenediamine hydrochloride (75 gm) is added to a mixture of methylene dichloride (500 ml) and triethylamine (25 gm) and stirred for 1 hour at 25 - 30°C. Then the reaction mass is added to a mixture of tetrahydro-2-furoic acid (54 gm), methylene dichloride (375 ml) and carbonyl diimidazole (75 gm) at 40 - 45°C and stirred for 3 hours at the same temperature. The reaction mass is then cooled to 20 - 25°C and the mass is filtered over hi-flo. To the filtrate is added water (500 ml) under stirring, the pH is adjusted to 12 using 10% NaOH solution, and washed twice with water. Then the organic layer is collected, washed with water (1000 ml) and then washed with NaCl solution (500 ml). The resulting organic layer is dried over sodium sulphate and distilled under vacuum to give oily residue (HPLC purity: 78.80%).

### Step-II:

The residue obtained in step-I is added to isopropyl alcohol (850 ml), cooled to 20°C and dry HCl gas is passed under stirring till the pH is reduced to 2. Then the resulting white solid is stirred for 1 hour at 25 - 30°C, the solid is filtered under N₂ atmosphere, washed with isopropyl alcohol (50 ml) and dried at 50°C for 3 hours to give 70 gm of alfuzosin hydrochloride (HPLC purity: 91%).

### Example 1

### Step-I:

Alfuzosin hydrochloride (70 gm, obtained in step-II of comparative example, HPLC purity: 91%) is added to a mixture of methylene dichloride (700 ml) and water (350 ml), and the pH is adjusted to 12 with 10% NaOH solution at 20 - 25°C. The contents are stirred for 15 minutes and the layers are separated. Then the aqueous layer is collected and re-extracted using methylene dichloride (350 ml). The organic layers are combined, washed with water (1000 ml) and then washed with 10% NaCl solution (500 ml). Then the organic layer is dried over sodium sulphate and distilled off the solvent under vacuum. Acetone (300 ml) is added and stirred for 1 hour 30 minutes at 40 - 45°C. Then the contents are cooled to 25 - 30°C and stirred for 2 hours. The solid is filtered, washed with acetone (50 ml) and then with diisopropyl ether (50 ml) under N₂ atmosphere, and dried at 50 - 55°C for 4 hours to give 60 gm of alfuzosin base (HPLC purity: 97%).

### Step-II:

The above alfuzosin base is suspended in acetone (300 ml), the suspension is stirred for 1 hour at 40 - 45°C and cooled to 20 - 25°C. Then the solid is filtered, washed with acetone (50 ml) and then with diisopropyl ether (50 ml), and dried at 50 - 55°C for 4 hours to give 50 gm of alfuzosin base (HPLC purity: 99.3%).

### Step-III:

The above alfuzosin base is added to acetone (500 ml), dry HCl gas is passed till the pH is reduced to 2 and stirred for 1 hour under N₂ atmosphere. Then the reaction mass is filtered under N₂ atmosphere, washed with acetone (50 ml) and dried at 55 - 60°C for 4 hours to give 37.5 gm of 99.3 % pure alfuzosin hydrochloride.

### Example 2

### Step-I:

Tetrahydro-2-furoic acid (54 gm) is dissolved in methylene dichloride (375 ml) at 25 - 30°C, cooled to 5 - 10°C and carbonyl diimidazole (75 gm) is added to the solution. The contents are stirred for 10 minutes, the temperature is raised to 40 - 45°C and maintained for 1 hour at the same temperature. Then the reaction mass is added to a mixture of N₁-(4-Amino-6,7-dimethoxyquinazol-2-yl)-N₁-methylpropylenediamine hydrochloride (75 gm), methylene dichloride (500 ml) and triethylamine (25 gm) at 40 - 45°C and maintained at the same temperature for 3 hours. The reaction mass is cooled to 20 - 25°C and the mass is filtered over hi-flo. To the filtrate is added water (500 ml) under stirring, the pH is adjusted to 12 using 10% NaOH solution, and washed twice with water. Then the organic layer is collected, washed with water (1000 ml) and then washed with NaCl solution (500 ml). The resulting organic layer is dried over sodium sulphate and distilled under vacuum to give oily residue (HPLC Purity: 79.8%).

### Step-II:

The above residue is suspended in acetone (300 ml), stirred for 30 minutes at 40 - 50°C and cooled to 25 - 30°C. Then the reaction mass is stirred for 4 hours at 25 - 30°C, the solid obtained is filtered, washed with acetone (50 ml) and dried for 4 hours at 50 - 55°C to give 60 gm of alfuzosin base (HPLC purity: 97%).

### Step-III:

The above alfuzosin base is added to acetone (300 ml) and stirred for 30 minutes at 50 - 55°C. The contents are cooled to 25 - 30°C and stirred for 2 hours. Then the solid obtained is filtered, washed with acetone (50 ml) and dried at 50 - 55°C for 4 hours to give 40 gm of alfuzosin base (HPLC purity: 99.56%). The Differential Scanning Calorimetry (DSC), Infra-red (IR) and x-Ray Powder diffraction spectrums of alfuzosin base is essentially same as those shown in Figures 1, 2 and 3 respectively.

### Step-IV:

Alfuzosin base obtained above is added to acetone (400 ml), dry HCl gas is passed till the pH of the reaction mass reaches 2 under N₂ atmosphere and stirred for 1 hour at 20 - 25°C. Then the reaction mass is filtered under N₂ atmosphere, washed with acetone (40 ml) and dried at 65 -70°C for 10 hours to give 40 gm of 99.5 % pure alfuzosin hydrochloride.

### Example 3

Oily residue (2.0 gm, HPLC purity: 79.8 %, obtained as in step-I of example 2) is added to methyl isobutyl ketone (100 ml) and heated to 80 - 85°C to form a clear solution. The solution is cooled to 25 - 30°C and stirred for 1 hour at the same temperature. Then the solution is cooled to 0 - 5°C and stirred for 1 hour at 0 - 5°C. Then the resulting solid is filtered and dried to give 1.0 gm of the 99.69% pure alfuzosin base. The Differential Scanning Calorimetry (DSC), Infra-red (IR) and x-Ray Powder diffraction spectrums of alfuzosin base is essentially same as those shown in Figures 1, 2 and 3 respectively.

### Example 4

Alfuzosin base (5 gm, HPLC purity: 97%, obtained as in step-II of example 2) is added to acetone (250 ml), heated to 55 - 60°C and stirred for 15 minutes at the same temperature to form a clear solution. The solution is filtered, removed the undissolved solids and the filtrate is stirred for 12 hours at 25 - 30°C. The reaction mass is cooled to 10 - 15°C and stirred for 2 hours at 10 - 15°C. Then the resulting solid is filtered and dried at 50 - 60°C for 4 hours to give 3 gm of 99.77% pure alfuzosin base. The Differential Scanning Calorimetry (DSC), Infra-red (IR) and x-Ray Powder diffraction spectrums of alfuzosin base obtained is shown in Figures 1, 2 and 3 respectively.

### Example 5

Alfuzosin base (5 gm, HPLC purity: 97%, obtained as in step-I of example 1) is added to methanol (55 ml) and heated to reflux to form a clear solution. The solution is cooled to 25 - 30°C and stirred for 12 hours at the same temperature. Then the solution is cooled to 10 - 15°C and stirred for 2 hours. The resulting solid is filtered, washed with methanol (5 ml) and dried at 50 - 60°C for 4 hours to give 3.5 gm of 99.95% pure alfuzosin base. The Differential Scanning Calorimetry (DSC), Infra-red (IR) and x-Ray Powder diffraction spectrums of alfuzosin base is essentially same as those shown in Figures 1, 2 and 3 respectively.

## Claims

1. Crystalline alfuzosin base.

2. Crystalline alfuzosin base of claim 1, wherein the purity is above 95%.

3. Crystalline alfuzosin base of claim 2, wherein the purity is above 99%.

4. A process for the preparation of crystalline alfuzosin base of claim 1, which comprises stirring a suspension of impure or noncrystalline alfuzosin base in a ketonic solvent or an alcoholic solvent or mixture thereof.

5. The process according to claim 4, further comprises the crystalline alfuzosin base obtained is collected by filtration or centrifugation.

6. The process according to claim 4, wherein the ketonic solvent is selected from acetone, methyl ethyl ketone, methyl isobutyl ketone, methyl isopropyl ketone and methyl tert-butyl ketone; and alcoholic solvent is selected from methanol, ethanol, isopropyl alcohol and tert-butyl alcohol.

7. The process according to claim 6, wherein the ketonic solvent is acetone or methyl isobutyl ketone.

8. The process according to claim 7, wherein the ketonic solvent is acetone.

9. The process according to claim 7, wherein the ketonic solvent is methyl isobutyl ketone.

10. The process according to claim 6, wherein the alcoholic solvent is methanol or ethanol.

11. The process according to claim 10, wherein the alcoholic solvent is methanol.

12. The process according to claim 4, wherein the suspension is stirred for at least 30 minutes at below boiling temperature of the solvent used.

13. The process according to claim 12, wherein the suspension is stirred for 1 hour to 4 hours at 25 - 60°C.

14. A process for the preparation of crystalline solid of alfuzosin base, which comprises dissolving alfuzosin base in a ketonic solvent or an alcoholic solvent or mixture thereof and crystallizing alfuzosin base from the solution.

15. The process according to claim 14, further comprises the crystalline alfuzosin base is collected by filtration or centrifugation.

16. The process according to claim 14, wherein the crystallization is initiated by a method such as cooling, seeding, partial removal of the solvent from the solution, by adding an anti-solvent to the solution or a combination thereof.

17. The process according to claim 14, wherein the ketonic solvent is selected from acetone, methyl ethyl ketone, methyl isobutyl ketone, methyl isopropyl ketone and methyl tert-butyl ketone; and alcoholic solvent is selected from methanol, ethanol, isopropyl alcohol and tert-butyl alcohol.

18. The process according to claim 17, wherein the ketonic solvent is acetone or methyl isobutyl ketone.

19. The process according to claim 18, wherein the ketonic solvent is acetone.

20. The process according to claim 18, wherein the ketonic solvent is methyl isobutyl ketone.

21. The process according to claim 17, wherein the alcoholic solvent is methanol or ethanol.

22. The process according to claim 21, wherein the alcoholic solvent is methanol.

23. A process for the preparation of crystalline solid of alfuzosin base, which comprises treating an acid addition salt of alfuzosin with a base to liberate alfuzosin base, isolating by forcible or spontaneous crystallization from a ketonic or an alcoholic solvent or mixture thereof.

24. The process according to claim 23, further comprises the crystalline alfuzosin base is collected by filtration or centrifugation.

25. The process according to claim 23, wherein forcible crystallization is initiated by a method such as cooling, seeding, partial removal of the solvent from the solution, by adding an anti-solvent to the solution or a combination thereof.

26. The process according to claim 23, wherein the ketonic solvent is selected from acetone, methyl ethyl ketone, methyl isobutyl ketone, methyl isopropyl ketone and methyl tert-butyl ketone; and alcoholic solvent is selected from methanol, ethanol, isopropyl alcohol and tert-butyl alcohol.

27. The process according to claim 26, wherein the ketonic solvent is acetone or methyl isobutyl ketone.

28. The process according to claim 27, wherein the ketonic solvent is acetone.

29. The process according to claim 27, wherein the ketonic solvent is methyl isobutyl ketone.

30. The process according to claim 26, wherein the alcoholic solvent is methanol or ethanol.

31. The process according to claim 30, wherein the alcoholic solvent is methanol.

32. The process according to claims 4, 14 and 23, which further comprises crystalline alfuzosin base is converted into a pharmaceutically acceptable salt of alfuzosin.

33. The process according to claim 32, wherein the pharmaceutically acceptable salt of alfuzosin is alfuzosin hydrochloride.

34. A process for the preparation of alfuzosin base or a pharmaceutically acceptable salt thereof, which comprises reacting N₁-(4-Amino-6,7-dimethoxyquinazol-2-yl)-N₁-methylpropylenediamine with activated tetrahydro-2-furoic acid by adding the said activated tetrahydro-2-furoic acid to the said diamine compound, isolating impure alfuzosin base from the reaction mixture, converting the said base into crystalline solid, optionally converting the said crystalline solid into pharmaceutically acceptable salt of alfuzosin.

35. The process according to claim 34, wherein the pharmaceutically acceptable salt is alfuzosin hydrochloride.

36. The process according to claims 34 and 35, wherein the impure alfuzosin base is converted into the said crystalline alfuzosin base by suspending the impure alfuzosin in a ketonic or an alcoholic solvent, stirring for at least 30 minutes at about 25 - 60°C, filtering or centrifuging, dissolving the obtained solid in an alcoholic or ketonic solvent, crystallizing and filtering to form crystalline alfuzosin base.

37. The process according to claim 36, wherein the solvent used for suspension is acetone.

38. The process according to claim 36, wherein the solvent used for dissolving alfuzosin base is methanol.

39. The process according to claims 34 and 35, wherein the impure alfuzosin is converted into crystalline solid by isolating as an acid addition salt, treating the salt with a base to liberate alfuzosin base, crystallizing from a ketonic or alcoholic solvent.

40. The process according to claim 39, wherein the acid addition salt is hydrochloride salt of alfuzosin, the solvent used for crystallization is methanol or acetone.

41. The process according to claims 34 and 35, wherein impure alfuzosin base is converted into the said crystalline alfuzosin by dissolving impure alfuzosin in a ketonic or an alcoholic solvent or a mixture thereof and crystallizing alfuzosin base from the solution.

42. The process according to claim 41, wherein the ketonic solvent is acetone or methyl isobutyl ketone and the alcoholic solvent is methanol or ethanol.

43. The process according to claims 34 and 35, wherein impure alfuzosin base is converted into the said crystalline alfuzosin by converting alfuzosin base into an acid addition salt thereof, isolating the salt obtained from the reaction mixture, reacting the salt with a base to liberate alfuzosin base, isolating alfuzosin base obtained from the reaction mixture, dissolving alfuzosin base in a ketonic solvent or an alcoholic solvent or mixture thereof and crystallizing alfuzosin base from the solution.

44. The process according to claim 43, wherein the acid addition salt is hydrochloride salt, the base is selected from hydroxides, carbonates or bicarbonates of sodium and potassium, the ketonic solvent is acetone or methyl isobutyl ketone and alcoholic solvent is methanol or ethanol.

45. The process according to claims 34 and 35, wherein impure alfuzosin base is converted into the said crystalline alfuzosin by converting alfuzosin base into an acid addition salt thereof, isolating the salt obtained from the reaction mixture, reacting the salt with a base to liberate alfuzosin base, isolating alfuzosin base obtained from the reaction mixture, suspending alfuzosin base in a ketonic solvent or an alcoholic solvent or mixture thereof for 1 hour to 4 hours at 25 - 60°C and collecting crystalline alfuzosin base by filtration or centrifugation.

46. The process according to claim 45, wherein the acid addition salt is hydrochloride salt, the base is selected from hydroxides, carbonates or bicarbonates of sodium and potassium, the ketonic solvent is acetone or methyl isobutyl ketone and alcoholic solvent is methanol or ethanol.

## Patentansprüche

1. Kristalliner Alfuzosingrundstoff.

2. Kristalliner Alfuzosingrundstoff nach Anspruch 1, wobei die Reinheit mehr als 95 % beträgt.

3. Kristalliner Alfuzosingrundstoff nach Anspruch 2, wobei die Reinheit mehr als 99 % beträgt.

4. Verfahren für die Herstellung von kristallinem Alfuzosingrundstoff nach Anspruch 1, das das Rühren einer Suspension von unreinem oder nichtkristallinem Alfuzosingrundstoff in einem ketonischen Lösungsmittel oder einem alkoholischen Lösungsmittel oder einer Mischung derselben umfasst.

5. Verfahren nach Anspruch 4, das des Weiteren umfasst, dass der erhaltene kristalline Alfuzosingrundstoff durch Filtrieren oder Zentrifugieren aufgefangen wird.

6. Verfahren nach Anspruch 4, wobei das ketonische Lösungsmittel unter Aceton, Methylethylketon, Methylisobutylketon, Methylisopropylketon und Methyl-tert.butylketon ausgewählt wird; und das alkoholische Lösungsmittel unter Methanol, Ethanol, Isoproylalkohol und tert.-Butylalkohol ausgewählt wird.

7. Verfahren nach Anspruch 6, wobei das ketonische Lösungsmittel Aceton oder Methylisobutylketon ist.

8. Verfahren nach Anspruch 7, wobei das ketonische Lösungsmittel Aceton ist.

9. Verfahren nach Anspruch 7, wobei das ketonische Lösungsmittel Methylisobutylketon ist.

10. Verfahren nach Anspruch 6, wobei das alkoholische Lösungsmittel Methanol oder Ethanol ist.

11. Verfahren nach Anspruch 10, wobei das alkoholische Lösungsmittel Methanol ist.

12. Verfahren nach Anspruch 4, wobei die Suspension mindestens 30 Minuten lang bei einer Temperatur unter der Siedetemperatur des verwendeten Lösungsmittels gerührt wird.

13. Verfahren nach Anspruch 12, wobei die Suspension 1 Stunde bis 4 Stunden lang bei 25 - 60 °C gerührt wird.

14. Verfahren für die Herstellung von kristallinem Feststoff aus Alfuzosingrundstoff, das das Lösen von Alfuzosingrundstoff in einem ketonischen Lösungsmittel oder alkoholischen Lösungsmittel oder einer Mischung derselben und das Kristallisieren des Alfuzosingrundstoffs aus der Lösung umfasst.

15. Verfahren nach Anspruch 14, das des Weiteren umfasst, dass der kristalline Alfuzosingrundstoff durch Filtrieren oder Zentrifugieren aufgefangen wird.

16. Verfahren nach Anspruch 14, wobei das Kristallisieren durch eine Methode wie Kühlen, Impfen, teilweises Entfernen des Lösungsmittels aus der Lösung, durch Hinzugeben eines Gegenlösungsmittels zur Lösung oder eine Kombination derselben initiiert wird.

17. Verfahren nach Anspruch 14, wobei das ketonische Lösungsmittel unter Aceton, Methylethylketon, Methylisobutylketon, Methylisopropylketon und Methyl-tert.butylketon ausgewählt wird; und das alkoholische Lösungsmittel unter Methanol, Ethanol, Isoproylalkohol und tert.-Butylalkohol ausgewählt wird.

18. Verfahren nach Anspruch 17, wobei das ketonische Lösungsmittel Aceton oder Methylisobutylketon ist.

19. Verfahren nach Anspruch 18, wobei das ketonische Lösungsmittel Aceton ist.

20. Verfahren nach Anspruch 18, wobei das ketonische Lösungsmittel Methylisobutylketon ist.

21. Verfahren nach Anspruch 17, wobei das alkoholische Lösungsmittel Methanol oder Ethanol ist.

22. Verfahren nach Anspruch 21, wobei das alkoholische Lösungsmittel Methanol ist.

23. Verfahren für die Herstellung von kristallinem Feststoff von Alfuzosingrundstoff, das das Behandeln eines sauren Additionssalzes von Alfuzosin mit einem Grundstoff zum Freisetzen des Alfuzosingrundstoffs, das Isolieren durch Zwangs- oder spontane Kristallisation aus einem ketonischen oder einem alkoholischen Lösungsmittel oder Mischungen desselben umfasst.

24. Verfahren nach Anspruch 23, das des Weiteren umfasst, dass der kristalline Alfuzosingrundstoff durch Filtrieren oder Zentrifugieren aufgefangen wird.

25. Verfahren nach Anspruch 23, wobei das Zwangskristallisieren durch eine Methode wie Kühlen, Impfen, teilweises Entfernen des Lösungsmittels aus der Lösung, durch Hinzugeben eines Gegenlösungsmittels zur Lösung oder eine Kombination derselben initiiert wird.

26. Verfahren nach Anspruch 23, wobei das ketonische Lösungsmittel unter Aceton, Methylethylketon, Methylisobutylketon, Methylisopropylketon und Methyl-tert.butylketon ausgewählt wird; und das alkoholische Lösungsmittel unter Methanol, Ethanol, Isoproylalkohol und tert.-Butylalkohol ausgewählt wird.

27. Verfahren nach Anspruch 26, wobei das ketonische Lösungsmittel Aceton oder Methylisobutylketon ist.

28. Verfahren nach Anspruch 27, wobei das ketonische Lösungsmittel Aceton ist.

29. Verfahren nach Anspruch 27, wobei das ketonische Lösungsmittel Methylisobutylketon ist.

30. Verfahren nach Anspruch 26, wobei das alkoholische Lösungsmittel Methanol oder Ethanol ist.

31. Verfahren nach Anspruch 30, wobei das alkoholische Lösungsmittel Methanol ist.

32. Verfahren nach den Ansprüchen 4, 14 und 23, das des Weiteren umfasst, dass der kristalline Alfuzosingrundstoff zu einem pharmazeutisch akzeptablen Salz von Alfuzosin umgewandelt wird.

33. Verfahren nach Anspruch 32, wobei das pharmazeutisch akzeptable Salz von Alfuzosin Alfuzosinhydrochlorid ist.

34. Verfahren für die Herstellung von Alfuzosingrundstoff oder eines pharmazeutisch akzeptablen Salzes desselben, das das Reagieren von N₁-(4-Amino-6,7-dimethoxychinazol-2-yl)-N₁-methylpropylendiamin mit aktivierter Tetrahydro-2-furonsäure durch Hinzugeben der aktivierten Tetrahydro-2-furonsäure zur Diaminverbindung, Isolieren des unreinen Alfuzosingrundstoffs von der Reaktionsmischung, Umwandeln des Grundstoffs zu kristallinem Feststoff, wahlweise Umwandeln des kristallinen Feststoffs zu pharmazeutisch akzeptablem Salz von Alfuzosin umfasst.

35. Verfahren nach Anspruch 34, wobei das pharmazeutisch akzeptable Salz Alfuzosinhydrochlorid ist.

36. Verfahren nach den Ansprüchen 34 und 35, wobei der unreine Alfuzosingrundstoff in den kristallinen Alfuzosingrundstoff durch Suspendieren des unreinen Alfuzosins in einem ketonischen oder einem alkoholischen Lösungsmittel, Rühren mindestens 30 Minuten lang bei etwa 25 - 60 °C, Filtrieren oder Zentrifugieren, Lösen des erhaltenen Feststoff in einem alkoholischen oder ketonischen Lösungsmittel, Kristallisieren und Filtrieren unter Bildung des kristallinen Alfuzosingrundstoffs umgewandelt wird.

37. Verfahren nach Anspruch 36, wobei das Lösungsmittel, das zum Suspendieren verwendet wird, Aceton ist

38. Verfahren nach Anspruch 36, wobei das Lösungsmittel, das zum Lösen des Alfuzosingrundstoffs verwendet wird, Methanol ist.

39. Verfahren nach den Ansprüchen 34 und 35, wobei das unreine Alfuzosin zu kristallinem Feststoff durch Isolieren als saures Additionssalz, Behandeln des Salzes mit eines Grundstoffs zum Freisetzen des Alfuzosingrundstoffs, Kristallisieren aus einem ketonischen oder alkoholischen Lösungsmittel umgewandelt wird.

40. Verfahren nach Anspruch 39, wobei das saure Additionssalz Hydrochloridsalz von Alfuzosin ist, das Lösungsmittel, das für das Kristallisieren verwendet wird, Methanol oder Aceton ist.

41. Verfahren den Ansprüchen 34 und 35, wobei der unreine Alfuzosingrundstoff zum kristallinen Alfuzosin durch Lösen des unreinen Alfuzosins in einem ketonischen oder alkoholische Lösungsmittel oder einer Mischung derselben und Kristallisieren des Alfuzosingrundstoffs aus der Lösung umgewandelt wird.

42. Verfahren nach Anspruch 41, wobei das ketonische Lösungsmittel Aceton oder Methylisobutylketon ist und das alkoholische Lösungsmittel Methanol oder Ethanol ist.

43. Verfahren nach den Ansprüchen 34 und 35, wobei unreiner Alfuzosingrundstoff zum kristallinem Alfuzosin durch Umwandeln des Alfuzosingrundstoffs zu einem sauren Additionssalz desselben, Isolieren des erhaltenen Salzes von der Reaktionsmischung, Reagieren des Salzes mit einem Grundstoff zum Freisetzen des Alfuzosingrundstoffs, Isolieren des erhaltenen Alfuzosingrundstoffs von der Reaktionsmischung, Lösen des Alfuzosingrundstoffs in einem ketonischen Lösungsmittel oder einem alkoholischen Lösungsmittel oder einer Mischung derselben und Kristallisieren des Alfuzosingrundstoffs aus der Lösung umgewandelt wird.

44. Verfahren nach Anspruch 43, wobei das saure Additionssalz Hydrochloridsalz ist, der Grundstoff unter Hydroxiden, Carbonaten oder Bicarbonaten von Natrium und Kalium ausgewählt wird, das ketonische Lösungsmittel Aceton oder Methylisobutylketon ist und das alkoholische Lösungsmittel Methanol oder Ethanol ist.

45. Verfahren nach Anspruch 34 und 35, wobei unreiner Alfuzosingrundstoff zum kristallinem Alfuzosin durch Umwandeln des Alfuzosingrundstoffs zu einem sauren Additionssalz desselben, Isolieren des erhaltenen Salzes von der Reaktionsmischung, Reagieren des Salzes mit eines Grundstoffs zum Freisetzen des Alfuzosingrundstoffs, Isolieren des erhaltenen Alfuzosingrundstoffs von der Reaktionsmischung, Suspendieren des Alfuzosingrundstoffs in einem ketonischen Lösungsmittel oder einem alkoholischen Lösungsmittel oder Mischungen derselben für 1 Stunde bis 4 Stunden bei 25 - 60°C und Auffangen des kristallinen Alfuzosingrundstoffs durch Filtrieren oder Zentrifugieren umgewandelt wird.

46. Verfahren nach Anspruch 45, wobei das saure Additionssalz Hydrochloridsalz ist, der Grundstoff unter Hydroxiden, Carbonaten oder Bicarbonaten von Natrium und Kalium ausgewählt wird, das ketonische Lösungsmittel Aceton oder Methylisobutylketon ist und das alkoholische Lösungsmittel Methanol oder Ethanol ist.

## Revendications

1. Alfuzosine base sous forme cristalline.

2. Alfuzosine base sous forme cristalline de la revendication 1, dont la pureté dépasse 95 %.

3. Alfuzosine base sous forme cristalline de la revendication 2, dont la pureté dépasse 99 %.

4. Procédé de préparation de l'alfuzosine base sous forme cristalline de la revendication 1, qui comprend l'étape consistant à soumettre à une agitation une suspension d'alfuzosine base impure ou non cristalline dans un solvant cétonique, un solvant alcoolique ou un mélange de ceux-ci.

5. Procédé selon la revendication 4, qui comprend également le recueil de l'alfuzosine base obtenue sous forme cristalline par filtration ou centrifugation.

6. Procédé selon la revendication 4, où le solvant cétonique est sélectionné parmi l'acétone, la méthyléthylcétone, la méthylisobutylcétone, la méthylisopropylcétone et la méthyl-tert-butylcétone ; et où le solvant alcoolique est sélectionné parmi le méthanol, l'éthanol, l'alcool isopropylique et l'alcool tert-butylique.

7. Procédé selon la revendication 6, où le solvant cétonique est l'acétone ou la méthylisobutylcétone.

8. Procédé selon la revendication 7, où le solvant cétonique est l'acétone.

9. Procédé selon la revendication 7, où le solvant cétonique est la méthylisobutylcétone.

10. Procédé selon la revendication 6, où le solvant alcoolique est le méthanol ou l'éthanol.

11. Procédé selon la revendication 10, où le solvant alcoolique est le méthanol.

12. Procédé selon la revendication 4, où la suspension est soumise à une agitation pendant au moins 30 minutes à une température inférieure à la température d'ébullition du solvant utilisé.

13. Procédé selon la revendication 12, où la suspension est soumise à une agitation pendant 1 à 4 heures à 25-60 °C.

14. Procédé de préparation de l'alfuzosine base sous forme cristalline solide, qui comprend la dissolution de l'alfuzosine base dans un solvant cétonique, un solvant alcoolique ou un mélange de ceux-ci et la cristallisation de l'alfuzosine base à partir de la solution.

15. Procédé selon la revendication 14, qui comprend également le recueil de l'alfuzosine base sous forme cristalline par filtration ou centrifugation.

16. Procédé selon la revendication 14, où la cristallisation est amorcée par une méthode telle qu'un refroidissement, un ensemencement, une élimination partielle du solvant de la solution, l'addition d'un antisolvant à la solution ou une combinaison de ces méthodes.

17. Procédé selon la revendication 14, où le solvant cétonique est sélectionné parmi l'acétone, la méthyléthylcétone, la méthylisobutylcétone, la méthylisopropylcétone et la méthyl-tert-butylcétone ; et où le solvant alcoolique est sélectionné parmi le méthanol, l'éthanol, l'alcool isopropylique et l'alcool tert-butylique.

18. Procédé selon la revendication 17, où le solvant cétonique est l'acétone ou la méthylisobutylcétone.

19. Procédé selon la revendication 18, où le solvant cétonique est l'acétone.

20. Procédé selon la revendication 18, où le solvant cétonique est la méthylisobutylcétone.

21. Procédé selon la revendication 17, où le solvant alcoolique est le méthanol ou l'éthanol.

22. Procédé selon la revendication 21, où le solvant alcoolique est le méthanol.

23. Procédé de préparation de l'alfuzosine base sous forme cristalline solide, qui comprend le traitement d'un sel d'addition d'acide de l'alfuzosine avec une base pour libérer l'alfuzosine base, qui est isolée par cristallisation forcée ou spontanée à partir d'un solvant cétonique ou alcoolique ou d'un mélange de ceux-ci.

24. Procédé selon la revendication 23, qui comprend également le recueil de l'alfuzosine base sous forme cristalline par filtration ou centrifugation.

25. Procédé selon la revendication 23, où la cristallisation forcée est amorcée par une méthode telle qu'un refroidissement, un ensemencement, une élimination partielle du solvant de la solution, l'addition d'un antisolvant à la solution ou une combinaison de ces méthodes.

26. Procédé selon la revendication 23, où le solvant cétonique est sélectionné parmi l'acétone, la méthyléthylcétone, la méthylisobutylcétone, la méthylisopropylcétone et la méthyl-tert-butylcétone ; et où le solvant alcoolique est sélectionné parmi le méthanol, l'éthanol, l'alcool isopropylique et l'alcool tert-butylique.

27. Procédé selon la revendication 26, où le solvant cétonique est l'acétone ou la méthylisobutylcétone.

28. Procédé selon la revendication 27, où le solvant cétonique est l'acétone.

29. Procédé selon la revendication 27, où le solvant cétonique est la méthylisobutylcétone.

30. Procédé selon la revendication 26, où le solvant alcoolique est le méthanol ou l'éthanol.

31. Procédé selon la revendication 30, où le solvant alcoolique est le méthanol.

32. Procédé selon les revendications 4, 14 et 23, qui comprend également la conversion de l'alfuzosine base sous forme cristalline en un sel pharmaceutiquement acceptable de l'alfuzosine.

33. Procédé selon la revendication 32, où le sel pharmaceutiquement acceptable de l'alfuzosine est le chlorhydrate d'alfuzosine.

34. Procédé de préparation de l'alfuzosine base ou d'un sel pharmaceutiquement acceptable de celle-ci, qui comprend la réaction de la N₁-(4-amino-6,7-diméthoxyquinazol-2-yl)-N₁-méthylpropylènediamine avec un acide tétrahydro-2-furoïque activé par l'addition dudit acide tétrahydro-2-furoïque activé au dit composé à fonction diamine, l'isolation de l'alfuzosine base impure du mélange réactionnel, la conversion de ladite base en un solide cristallin et, en option, la conversion dudit solide cristallin en un sel pharmaceutiquement acceptable de l'alfuzosine.

35. Procédé selon la revendication 34, où le sel pharmaceutiquement acceptable de l'alfuzosine est le chlorhydrate d'alfuzosine

36. Procédé selon les revendications 34 et 35, où l'alfuzosine base impure est convertie en ladite alfuzosine base sous forme cristalline par une mise en suspension de l'alfuzosine impure dans un solvant cétonique ou alcoolique, un maintien sous agitation pendant au moins 30 minutes à environ 25-60 °C, une filtration ou une centrifugation, la dissolution du solide obtenu dans un solvant cétonique ou alcoolique, une cristallisation et une filtration pour former l'alfuzosine base sous forme cristalline.

37. Procédé selon la revendication 36, où le solvant utilisé pour la mise en suspension est l'acétone.

38. Procédé selon la revendication 36, où le solvant utilisé pour la dissolution de l'alfuzosine base est le méthanol.

39. Procédé selon les revendications 34 et 35, où l'alfuzosine impure est convertie en un solide cristallin par une isolation sous forme d'un sel d'addition d'acide, un traitement du sel par une base pour libérer l'alfuzosine base et une cristallisation à partir d'un solvant cétonique ou alcoolique.

40. Procédé selon la revendication 39, où le sel d'addition d'acide est le chlorhydrate d'alfuzosine et le solvant utilisé pour la cristallisation le méthanol ou l'acétone.

41. Procédé selon les revendications 34 et 35, où l'alfuzosine base impure est convertie en ladite alfuzosine sous forme cristalline par une dissolution de l'alfuzosine impure dans un solvant cétonique ou alcoolique ou un mélange de ceux-ci et une cristallisation de l'alfuzosine base à partir de la solution.

42. Procédé selon la revendication 41, où le solvant cétonique est l'acétone ou la méthylisobutylcétone et le solvant alcoolique est le méthanol ou l'éthanol.

43. Procédé selon les revendications 34 et 35, où l'alfuzosine base impure est convertie en ladite alfuzosine sous forme cristalline par une conversion de l'alfuzosine base en un sel d'addition d'acide de celle-ci, l'isolation du mélange réactionnel du sel obtenu, la réaction du sel avec une base pour libérer l'alfuzosine base, l'isolation du mélange réactionnel de l'alfuzosine base obtenue, la dissolution de l'alfuzosine base dans un solvant cétonique, un solvant alcoolique ou un mélange de ceux-ci et la cristallisation de l'alfuzosine base à partir de la solution.

44. Procédé selon la revendication 43, où le sel d'addition d'acide est le chlorhydrate, la base est sélectionnée parmi des hydroxydes, carbonates ou bicarbonates de sodium et de potassium, le solvant cétonique est l'acétone ou la méthylisobutylcétone et le solvant alcoolique est le méthanol ou l'éthanol.

45. Procédé selon les revendications 34 et 35, où l'alfuzosine base impure est convertie en ladite alfuzosine sous forme cristalline par une conversion de l'alfuzosine base en un sel d'addition d'acide de celle-ci, l'isolation du mélange réactionnel du sel obtenu, la réaction du sel avec une base pour libérer l'alfuzosine base, l'isolation du mélange réactionnel de l'alfuzosine base obtenue, la mise en suspension de l'alfuzosine base dans un solvant cétonique ou alcoolique ou un mélange de ceux-ci pendant 1 à 4 heures à 25-60 °C, et le recueil de l'alfuzosine base sous forme cristalline par filtration ou centrifugation.

46. Procédé selon la revendication 45, où le sel d'addition d'acide est le chlorhydrate, la base est sélectionnée parmi des hydroxydes, carbonates ou bicarbonates de sodium et de potassium, le solvant cétonique est l'acétone ou la méthylisobutylcétone et le solvant alcoolique est le méthanol ou l'éthanol.
